# EUROPEAN PATENT SPECIFICATION

(11) **EP 3 048 108 B1**
(45) Date of publication and mention of the grant of the patent: **15.07.2020**
(21) Application number: 14845625.4
(22) Date of filing: 10.09.2014
(51) Int. Cl.: C07F 9/6561, C07D 495/04, A61K 31/675, A61K 31/4535, A61P 7/02, A61P 9/04, A61P 9/10

(54) **THIENOPIPERIDINE DERIVATIVE AND USE THEREOF**
THIENOPIPERIDINDERIVAT UND VERWENDUNG DAVON
DÉRIVÉ THIÉNOPIPÉRIDINE ET SON UTILISATION

(30) Priority: 17.09.2013 CN 201310428052
(43) Date of publication of application: 27.07.2016
(73) Proprietor: Jiangsu Tasly Diyi Pharmaceutical Co., Ltd., Huai'an, Jiangsu 223003 (CN)
(72) Inventor: WANG, Guocheng, Tianjin 300410 (CN); ZHONG, Jun, Tianjin 300410 (CN)
(74) Representative: Gulde & Partner
(86) International application number: PCT/CN2014/086191
(87) International publication number: WO 2015/039577

(56) References cited:
- EP-A1- 2 532 668
- WO-A1-2012/025942
- WO-A1-2014/043895
- CN-A- 102 002 053
- CN-A- 102 993 210
- CN-A- 103 665 042
- JARKKO RAUTIO ET AL: "Prodrugs: design and clinical applications", NATURE REVIEWS. DRUG DISCOVERY, vol. 7, no. 3, 1 February 2008 (2008-02-01), pages 255-270, XP055227338, GB ISSN: 1474-1776, DOI: 10.1038/nrd2468

## Description

### Field of the invention

The present invention relates to organic chemical and medicinal chemical area. More specifically, the present invention relates to thienopiperidine derivatives and pharmaceutically acceptable acid addition salt thereof. The present invention also relates to the use of the thienopiperidine derivatives and pharmaceutically acceptable acid addition salt thereof in preparing drugs for preventing platelet aggregation and for treating and preventing cardiovascular and cerebrovascular diseases.

### Background of the invention

Clopidogrel is one kind of thienopiperidine derivative medicine, which could efficiently inhibit the platelet activity, is one anti-platelet medicine widely used for acute coronary syndrome and patients treated with percutaneous coronary intervention, with the following structural formula :

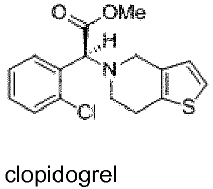

Clopidogrel is one kind of prodrug with no activity, which needs to be converted into active metabolite by liver cytochrome P450 (CYP450), the metabolic process of which is as follows:

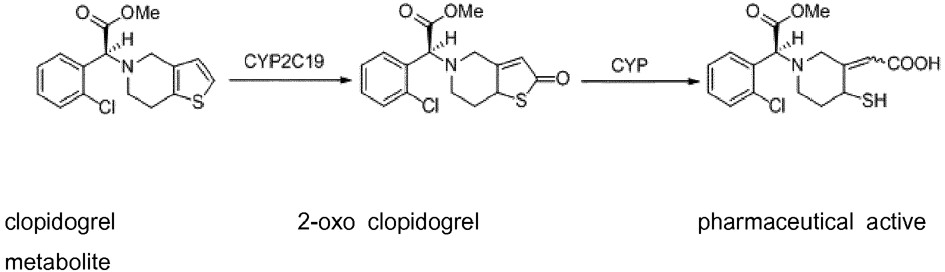

The metabolite binds with adenosine diphosphate (ADP) receptor P2Y12 on platelet membrane surface, playing a role of blocking the binding of ADP and platelet receptor and secondary ADP-mediated glycoprotein GPIIbPIIIa complex activation, and then inhibiting platelet aggregation (Arterioscler. Thromb. Vase. Biol., 1999, 19 (8): 2002-2011). Clopidogrel can significantly lower the occurrence rate of subacute stent thrombosis, decrease the occurrence of death, recurrent myocardial infarction and other cardiovascular events. However, recent research has found that about 11%∼44% (Am. Heart J., 2009, 157(2): 375-382) patients show low response even no response to clopidogrel, which is defined as clopidogrel resistance.

Exemplary clopidogrel derivatives are disclosed in EP2532668 A1, CN 103665042 A, WO 2014/043895 A1, and WO 2012/025942 A1.

Therefore, there's a need to develop a new antiplatelet drug which has fast action, high efficacy and can avoid clopidogrel resistance on clinic. Meanwhile, finding a compound which is favorable for preparation, in order to improve bioavailability, reduce side effect, and be favorable for dissolution, absorption and administration.

### Summary of the invention

The object of the present invention is to provide a new thienopiperidine derivative which acts as a prodrug of clopidogrel metabolite 2-oxo clopidogrel, to develop an antiplatelet drug with fast action and high bioavailability.

More specially, one object of the present invention is to provide an optical active thienopiperidine derivative or pharmaceutically acceptable salt thereof as recited in claim 1.

Another object of the present invention is to provide a pharmaceutical composition as recited in claim 3 with the optical active thienopiperidine derivative or pharmaceutically acceptable salt thereof as active constituents.

Another object of the present invention is to provide the optical active thienopiperidine derivative or pharmaceutically acceptable salt thereof as recited in claim 5 or pharmaceutical composition as recited in claim 8 for use as a medicament.

Another object of the present invention is to provide the uses of the optical active thienopiperidine derivative or pharmaceutically acceptable salt thereof as recited in claim 6 or pharmaceutical composition as recited in claim 9 for use in the treatment or preventive treatment of cardiovascular and cerebrovascular diseases.

That is, claim 1 refers to:
A thienopiperidine derivative or pharmaceutically acceptable acid addition salts thereof, wherein the thienopiperidine derivative is selected from the group consisting of:

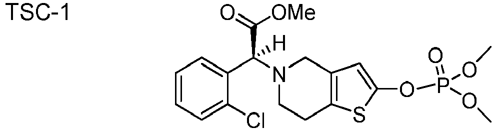

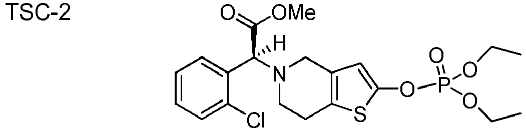

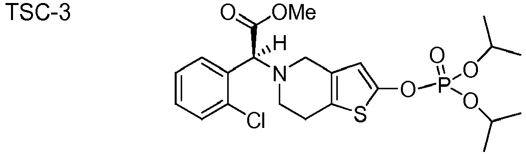

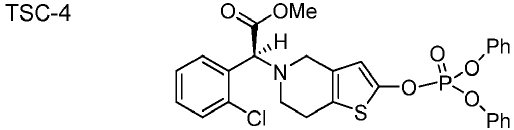

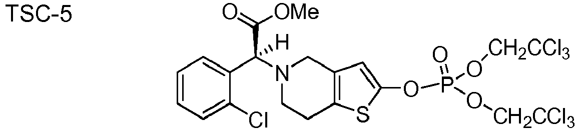

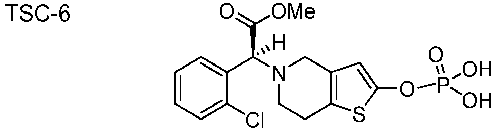

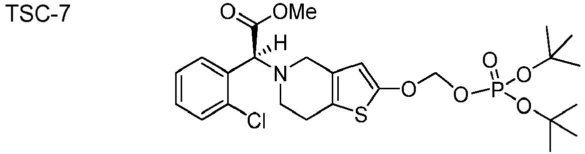

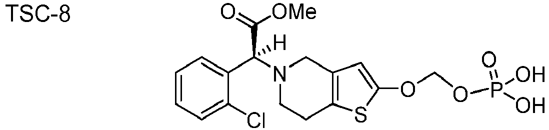

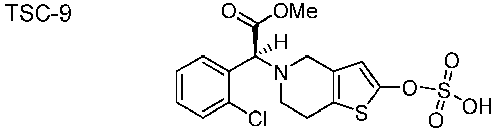

### Detailed description

To achieve the above objects, the present invention adopts the following technical scheme:

The present disclosure provides an optical active thienopiperidine derivative of a formula (I) and pharmaceutically acceptable salts thereof or a pharmaceutical composition comprising the compounds as active constituents:

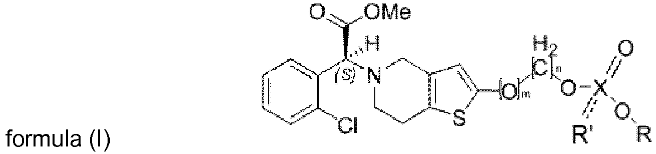

In formula (I), 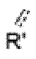 can be -O-R or =O, that is, the compounds according to formula (I) in the present disclosure can be represented by formula (II) and formula (III):

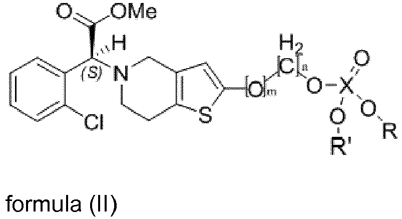

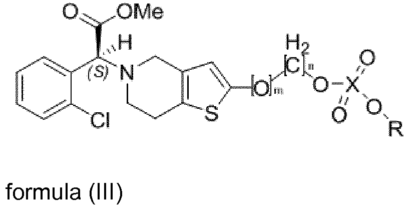

In formula (I) to formula (III),
X is P or S; m is 0 or 1; n is 0 or 1; R, R' can be the same or different, respectively and independently are H, C₁-C₄ straight or branched alkyl substituted by halogen or unsubstituted, phenyl or substituted phenyl.

Preferably, X is P; m is 0; n is 0; R, R' can be the same or different, respectively and independently are H, CH₃-, CH₃CH₂-, propyl, CCl₃CH₂- or phenyl; more preferably, the propyl is isopropyl.

Or, preferably, X is P; m is 1; n is 1; R, R' can be the same or different, respectively and independently are H, CH₃-, CH₃CH₂-, propyl, CCl₃CH₂-, butyl or phenyl; more preferably, the propyl is isopropyl, the butyl is tert-butyl.
Or, preferably, X is S; m is 0; n is 0; R is H, CH₃-, CH₃CH₂-, propyl, CCl₃CH₂-, butyl or phenyl; more preferably, the propyl is isopropyl, the butyl is tert-butyl.

The thienopiperidine derivatives of the present invention are represented by the following compounds:

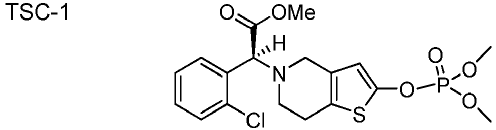

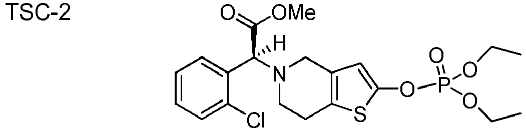

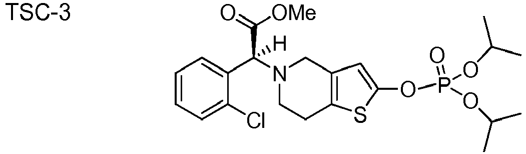

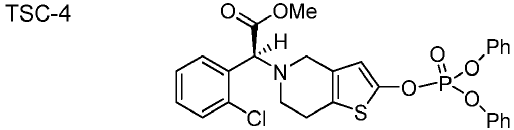

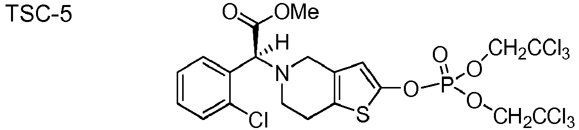

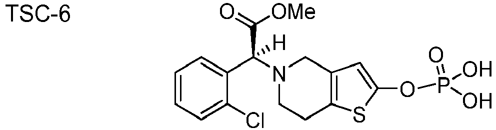

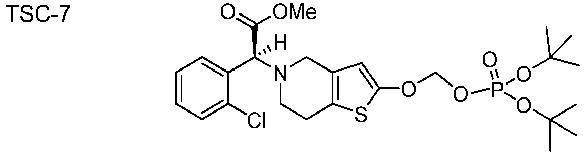

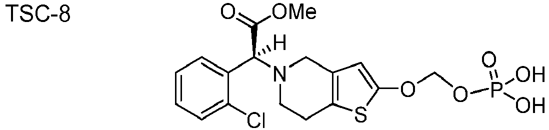

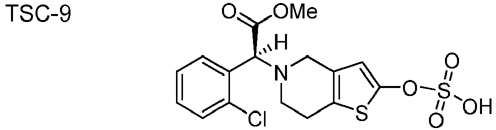

As another side of the invention, pharmaceutically acceptable acid addition salts of the thienopiperidine derivative are also included, wherein said acceptable acid addition salts are prepared by reacting the thienopiperidine derivative with the following organic acid or inorganic acid: sulphuric acid, muriatic acid, hydrobromic acid, phosphoric acid, tartaric acid, fumaric acid, maleic acid, citric acid, acetic acid, formic acid, methanesulfonic acid, p-toluene sulfonic acid, oxalic acid or succinic acid and so on.

As another side of the present disclosure, a method for preparing thienopiperidine derivative or pharmaceutically acceptable acid addition salts thereof is provided.

For example, for the thienopiperidine derivative according to formula (II) of the present disclosure, the preparation methods are as follows:
While m=0, n=0, 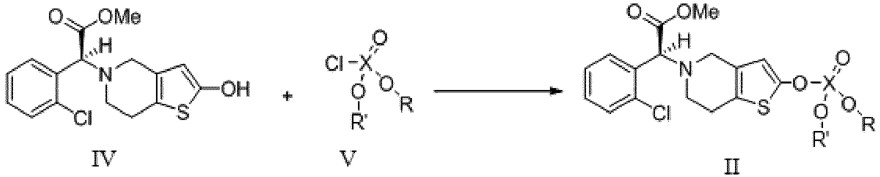
While m=1, n=1: 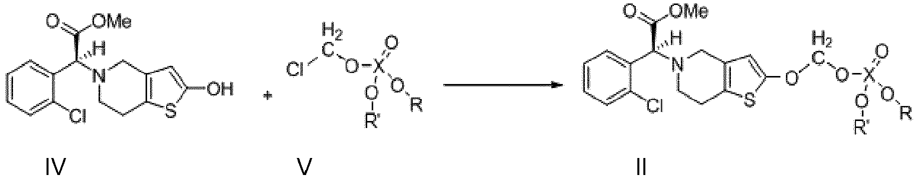 Wherein, the definition of the substituents is as mentioned above.

For the thienopiperidine derivative according to formula (III) in the present disclosure, its preparation methods are as follows:
While m=0, n=0: 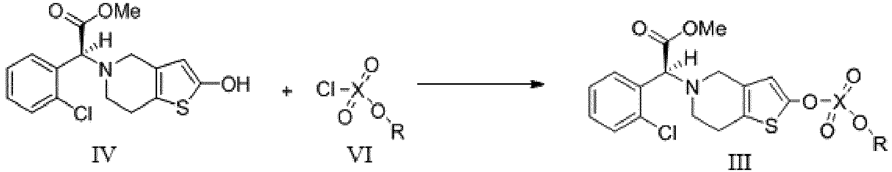
While m=1, n=1: 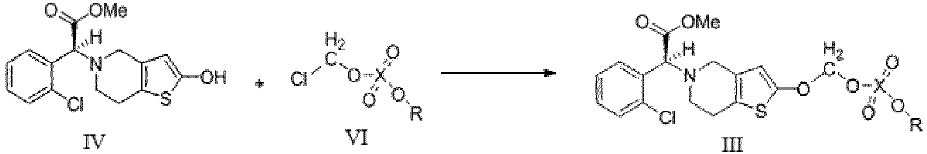 wherein the definition of the substituents is as mentioned above. Besides, the formula VI can also be replaced by sodium salts.

More specially, according to the detailed description of the present invention, the compound TSC-9 can be prepared by the following method: 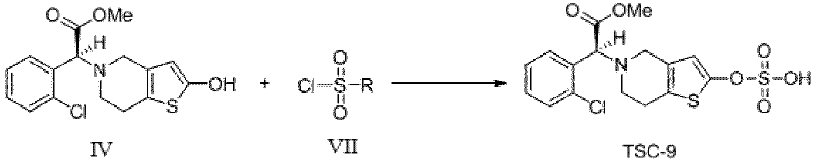 Wherein, R is chlorine or hydroxyl.

The method for preparing the material compound according to formula (IV) can refer to literature Journal of Medicinal Chemistry, 2012, 55(7), 3342-3352.

As another side of the present invention, it also provides a pharmaceutical composition, wherein said composition comprises the thienopiperidine derivative or pharmaceutically acceptable acid addition salts thereof as active constituents. As required, the pharmaceutical composition can also comprise pharmaceutically acceptable carrier(s). The pharmaceutically acceptable carrier(s) can be solid or liquid.

The pharmaceutical composition of the present invention can be made into solid or semisolid pharmaceutical preparations in the form of powder (like dispersible powder), tablet, capsule, suppository, plaster, gelata and so on, in this case, solid carriers are usually used. The solid carriers are preferably chosen from one or more of diluent, flavouring agent, solubilizer, lubricant, suspension concentrate, adhesive, expander, pharmacoat and so on. In powder preparation, the carrier contains 5wt%-70wt% of micronized active constituents. The instantiations of suitable solid carriers include magnesium carbonate, magnesium stearate, talc, sucrose, lactose, pectin, dextrine, starch, gelatin, tragacanth gum, methyl cellulose, carboxymethylcellulose sodium, low boiling wax, cacao butter and so on. The solid or semisolid pharmaceutical preparation is easy for drug administration, so it's a preferable preparation form, especially the solid preparation represented by tablet, powder, capsule are the oral solid preparation mostly favourable to be absorbed.

Besides, the pharmaceutical composition of the present invention can also be made into liquid preparation. The liquid preparation includes solution, injection, suspension concentrate and emulsion. For example, injection for non-parenteral administration can be made in the form of aqueous solution, propylene glycol aqueous solution or polyethylene glycol aqueous solution, the injection's isotonic concentration, pH and so on are adjusted, making it suitable for the physiological condition of the living body. For another example, the above active constituents can be dissolved in the water, and then suitable colorant, flavouring agent, stabilizer and thickener are added, to prepare oral solution; or, the micronized active constituents can be dispersed in the goop (like natural or synthetic rubber), methyl cellulose, carboxymethylcellulose sodium and other known suspending medium, to prepare oral suspension concentrate.

For easy drug administration and uniform dosage, it's very favourable to prepare the above pharmaceutical preparation in dosage unit form. The dosage unit form is a physical separation unit suitable to be a single dosage, each unit contains predetermined amount of active constituents producing the desired therapeutic effect. The dosage unit form can be in the form of package, like tablet, capsule or powder in small tubules or bottles, or ointment, gelata or cream in tubules or bottles.

Although the amount of the active constituents in each dosage unit form can be changed, it's usually regulated in the range of 1-1000mg, according to the effectiveness of the chosen active constituents.

A person skilled in the art can determine the preferable dosage suitable for some situation according to the regular methods. Generally speaking, the dosage at the beginning of treatment is lower than the optimal dosage of the active constituents, and then the drug administration dosage is increased gradually, until the best therapeutic effect is achieved. For convenience, the total daily dosage can be divided into several parts, several times to administer drugs.

As another side of the present invention, the present invention relates to the uses of the thienopiperidine derivative or pharmaceutically acceptable acid addition salt thereof in preparing drugs for treating or preventing cardiovascular and cerebrovascular diseases including heart failure, apoplexy, unstable angina and so on, especially the uses in preparing drugs for preventing platelet aggregation.

The beneficial effects of the present invention is that, the present invention provides a new kind of compound preventing platelet aggregation obviously, which is the prodrug of clopidogrel metabolite 2-oxo clopidogrel, can be metabolized into 2-oxo clopidogrel without CYP2C19 enzyme in vivo, having fast action, high efficacy, besides, the present invention is hoped to solve the problem of clopidogrel resistance due to the expression difference of P450 (cytochrome P450, CYP) enzyme in different individuals.

### Detailed description of the invention

The below, further explains the present invention according to examples.

### Preparation example 1

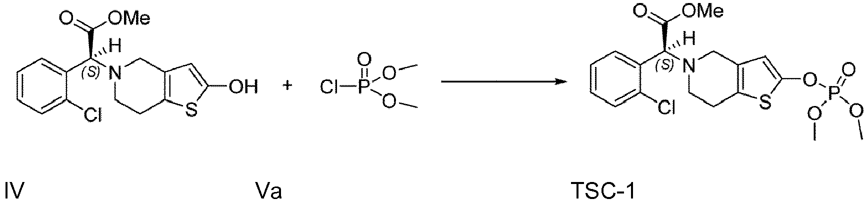

2-oxo clopidogrel intermediate IV (200mg, 0.6 mmol) was dissolved in 5ml anhydrous tetrahydrofuran, cooled to minus 20 degrees, lithium diisopropylamide (2.0M, 0.5 ml, 1 mmol) was added and stirred for 20 minutes, compound Va (104 mg, 0.72 mmol) was added into the reaction solution, raised naturally to room temperature to react for 12 hours, quenched by 4% hydrochloric acid, 50ml ethyl acetate was added, the organic layer was washed by sodium bicarbonate and saturated salt water respectively, dried with anhydrous sodium sulfate, filtrated and concentrated. After purification by silica gel column chromatograph (petroleum ether: ethyl acetate (PE:EA) = 4:1), compound TSC-1 (245mg, yield 92%) was obtained.
¹H NMR(400 MHz, CDCl₃): δ 7.67-7.65 (m, 1H), 7.42-7.40 (m, 1H), 7.31-7.26 (m, 2H), 6.25 (d, 1H), 4.91 (s, 1H), 3.87 (s, 3H), 3.72 (s, 3H), 3.64-3.60 (m, 1H), 3.51-3.48 (m, 1H), 2.89-2.87 (m, 2H), 2.75-2.73 (m, 2H), MS: m/z 446 [M+1]⁺.

### Preparation example 2

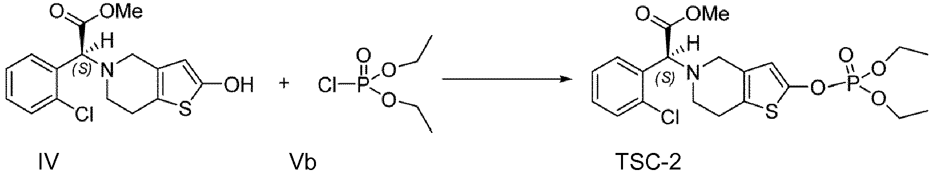

2-oxo clopidogrel intermediate IV (500mg, 1.5 mmol) was dissolved in 10ml anhydrous tetrahydrofuran, cooled to minus 20 degrees, lithium diisopropylamide (2.0M, 1.25 ml, 2.5 mmol) was added and stirred for 30 minutes, compound Vb (311 mg, 1.8 mmol) was added into the reaction solution, raised naturally to room temperature to react for 12 hours, quenched by 4% hydrochloric acid, 100ml ethyl acetate was added, the organic layer was washed by sodium bicarbonate and saturated salt water respectively, dried with anhydrous sodium sulfate, filtrated and concentrated. After purification by silica gel column chromatograph (PE:EA= 4:1), compound TSC-2 (660mg, yield 93%) was obtained.
¹H NMR(400 MHz, CDCl₃): δ 7.69-7.66 (m, 1H), 7.43-7.41 (m, 1H), 7.33-7.28 (m, 2H), 6.27 (d, 1H), 4.91 (s, 1H), 4.27-4.18 (m, 4H), 3.73 (s, 3H), 3.65-3.61 (m, 1H), 3.52-3.49 (m, 1H), 2.90-2.87 (m, 2H), 2.76-2.74 (m, 2H), 1.39-1.36 (dt, 6H). MS: m/z 474 [M+1]⁺.

### Preparation example 3

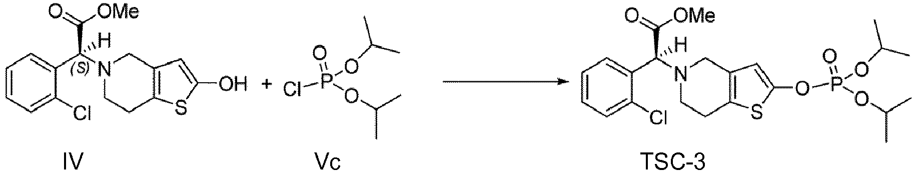

2-oxo clopidogrel intermediate IV (150mg, 0.45 mmol) was dissolved in 5ml anhydrous tetrahydrofuran, cooled to minus 20 degrees, lithium diisopropylamide (2.0M, 0.4 ml, 0.8 mmol) was added and stirred for 20 minutes, compound Vc (108 mg, 0.54 mmol) was added into the reaction solution, raised naturally to room temperature to react for 12 hours, quenched by 4% hydrochloric acid, 50ml ethyl acetate was added, the organic layer was washed by sodium bicarbonate and saturated salt water respectively, dried with anhydrous sodium sulfate, filtrated and concentrated. After purification by silica gel column chromatograph (PE:EA= 2:1), compound TSC-3 (192mg, yield 85%) was obtained.
¹H NMR(400 MHz, CDCl₃): δ 7.68-7.67 (m, 1H), 7.41-7.39 (m, 1H), 7.34-7.28 (m, 2H), 6.28 (d, 1H), 4.92 (s, 1H), 4.74 (m, 2H), 4.26-4.17 (m, 4H), 3.73 (s, 3H), 3.64-3.61 (m, 1H), 3.53-3.49 (m, 1 H),1.28 (d, 12H). MS: m/z 502 [M+1]⁺.

### Preparation example 4

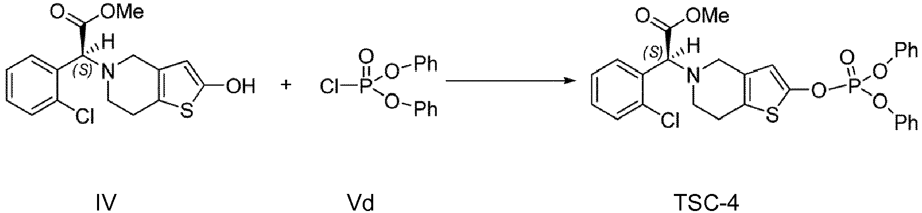

2-oxo clopidogrel intermediate IV (100mg, 0.3 mmol) was dissolved in 5ml anhydrous tetrahydrofuran, cooled to minus 20 degrees, lithium diisopropylamide (2.0M, 0.25 ml, 0.5 mmol) was added and stirred for 20 minutes, compound Vd (97 mg, 0.36 mmol) was added into the reaction solution, raised naturally to room temperature to react for 12 hours, quenched by 4% hydrochloric acid, 50ml ethyl acetate was added, the organic layer was washed by sodium bicarbonate and saturated salt water respectively, dried with anhydrous sodium sulfate, filtrated and concentrated. After purification by silica gel column chromatograph (PE:EA= 2:1), compound TSC-4 (162mg, yield 95%) was obtained.
¹H NMR(400 MHz, CDCl₃): δ 7.71-7.68 (m, 1H), 7.47-7.42 (m, 5H), 7.35-7.24 (m, 10H), 6.28 (d, 1H), 4.92 (s, 1H), 3.64-3.60 (m, 1H), 3.51-3.48 (m, 1H), 2.89-2.87 (m, 2H), 2.75-2.73 (m, 2H), MS: m/z 570 [M+1]⁺.

### Preparation example 5

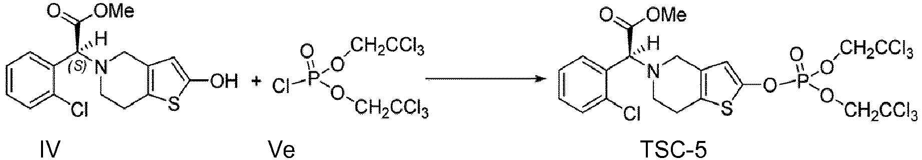

2-oxo clopidogrel intermediate IV (300mg, 0.9 mmol)was dissolved in 15ml anhydrous tetrahydrofuran, cooled to minus 20 degrees, lithium diisopropylamide (2.0M, 0.75 ml, 1.5 mmol)was added and stirred for 20 minutes, compound Ve (493 mg, 1.3 mmol) was added into the reaction solution, raised naturally to room temperature to react for 12 hours, quenched by 4% hydrochloric acid, 200ml ethyl acetate was added, the organic layer was washed by sodium bicarbonate and saturated salt water respectively, dried with anhydrous sodium sulfate, filtrated and concentrated. After purification by silica gel column chromatograph (PE:EA= 3:1), compound TSC-5 (400mg, yield 65%) was obtained.
¹H NMR(400 MHz, CDCl₃): δ 7.68-7.67 (m, 1H), 7.41-7.39 (m, 1H), 7.34-7.28 (m, 2H), 6.28 (d, 1H), 4.92 (s, 1H), 4.26-4.17 (m, 4H), 3.73 (s, 3H), 3.64-3.61 (m, 1H), 3.53-3.49 (m, 1H), 2.92-2.88 (m, 2H), 2.76-2.75 (m, 2H). MS: m/z 678 [M+1]⁺.

### Preparation example 6

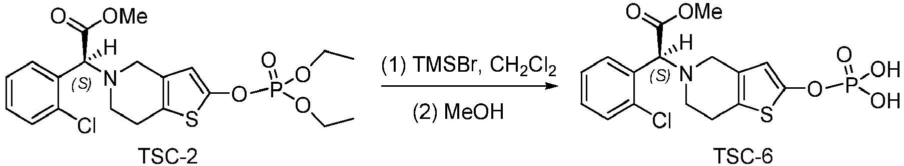

TSC-2 (500 mg, 1.04 mmol) was dissolved in 10 ml dry dichloromethane, TMSBr (1.7 ml, 13 mmol) was added, reacted at room temperature for 12h, the reaction was stopped, the solvent was removed under reduced pressure, 10ml methanol was added and stirred for 1h. The reaction solution was concentrated directly, purified by silica gel column chromatograph (n-butanol:formic acid:water=5:5:1), compound TSC-6 (390mg, yield 90%) was obtained.
¹H NMR(400 MHz, DMSO): δ 7.60 (d, 1H), 7.53 (d, 1H), 7.41-7.40 (m, 2H), 6.24 (s, 1H), 4.91 (s, 1H), 3.67 (s, 3H), 3.56 (s, 2H), 2.85 (brs, 2H), 2.66 (brs, 2H), MS: m/z 418 [M+1]⁺.

### Preparation example 7

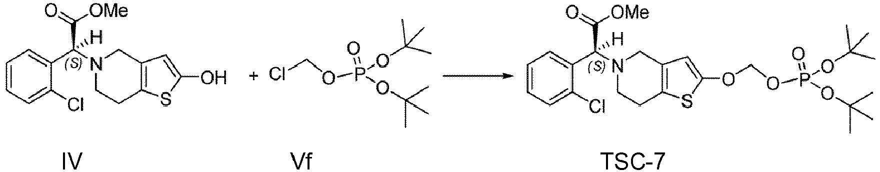

2-oxo clopidogrel intermediate IV (500mg, 1.5 mmol) was dissolved in 5ml anhydrous tetrahydrofuran, cooled to minus 20 degrees, lithium diisopropylamide (2.0M, 1.25 ml, 2.5 mmol) was added and stirred for 20 minutes, compound Vf (466 mg, 1.8 mmol) was added into the reaction solution, raised naturally to room temperature to react for 12 hours, quenched by 4% hydrochloric acid, 100ml ethyl acetate was added, the organic layer was washed by sodium bicarbonate and saturated salt water respectively, dried with anhydrous sodium sulfate, filtrated and concentrated. After purification by silica gel column chromatograph (PE:EA = 2:1), compound TSC-7 (269mg, yield 32%) was obtained.
¹H NMR(400 MHz, CDCl₃): δ 7.69-7.65 (m, 1H), 7.42-7.40 (m, 1H), 7.31-7.24 (m, 2H), 6.17 (s, 1H), 5.46 (s, 1H), 5.43 (s, 1H), 4.91 (s, 1H), 3.73 (s, 3H), 3.64-3.60 (m, 1H), 3.50-3.47 (m, 1H), 2.91-2.88 (m, 2H), 2.75-2.72 (m, 2H), 1.50 (s, 18H). MS: m/z 560 [M+1]⁺.

### Preparation example 8

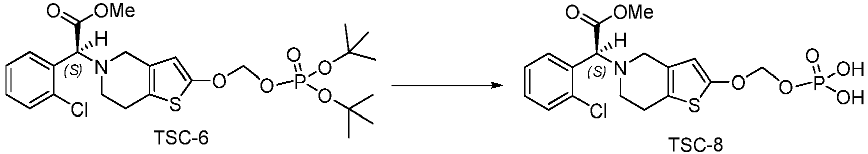

TSC-6 (500mg, 0.89 mmol)was dissolved in 10ml dichloromethane, trifluoroacetic acid (2ml) was added, stirred at room temperature for 1h, concentrated under reduced pressure, purified by silica gel column chromatograph (n-butanol:formic acid:water=5:5:1), compound TSC-8 (140mg, yield 35%) was obtained.
¹H NMR(400 MHz, DMSO): δ 7.62-7.60 (m, 1H), 7.54-7.41 (m, 3H), 6.18 (s, 1H), 5.84 (s, 1H), 5.37-5.32 (d, 2H), 4.26-3.98 (m, 2H), 3.79 (s, 3H), 3.74-3.66 (m, 2H), 3.15-3.00 (m, 2H), MS: m/z 448 [M+1]⁺.

### Preparation example 9

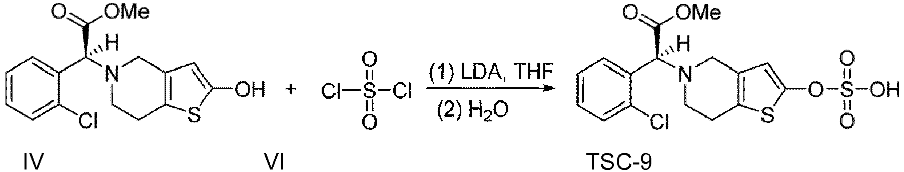

2-oxo clopidogrel intermediate IV (500mg, 1.5 mmol) was dissolved in 5ml anhydrous tetrahydrofuran, cooled to minus 20 degrees, lithium diisopropylamide (LDA, 2.0M, 1.25 ml, 2.5 mmol) was added and stirred for 20 minutes, compound VI was added into the reaction liquid, raised naturally to room temperature to react for 12 hours, quenched by 4% hydrochloric acid, 100ml ethyl acetate was added, the organic layer was washed by sodium bicarbonate and saturated salt water respectively, dried with anhydrous sodium sulfate, filtrated and concentrated. After purification by silica gel column chromatograph (PE:EA = 2:1), compound TSC-9 (269mg, yield 32%) was obtained.
¹H NMR(400 MHz, CDCl₃): δ 7.59 (s, 1H), 7.39-7.37 (m, 1H), 7.27-7.26 (d, 2H), 6.50 (brs, 1H), 6.34 (s, 1H), 4.97 (s, 1H), 3.68-3.58 (m, 5H), 2.90-2.73 (m, 4H); MS: m/z 418 [M+1]⁺.

### Experiment example 1 Research on pharmacodynamics of the compound in the present invention

### Methods

A small dosage of ADP (with a concentration less than 0.9 µmol/l) was added in the platelet suspension, which could cause platelet aggregation quickly, but then deaggregation; if a medium dosage of ADP (about 1.0µmol/l)was added, a second irreversible condensed phase appeared after the first condensed phase ended and soon after the deaggregation. The maximum aggregation rate of irreversible condensed phase can be used to evaluate the effect of subject products on coagulation function. The experiment used NJ4 type Semi-Platelet Aggregation Analyser of precil company, to survey the inhibitory effect of the subject products provided by Tasly Holding Group.Co.Ltd on platelet aggregation.

**Materials**

| | |
|---|---|
| Animals: | male Wistar rats, body weight 230-250g, bought from Beijing Vital River Laboratory Animal Technology Co. Ltd., animal certification number: SCXK 2007-0001. |
| Reagents: | ADP, bought from Sigma company; clopidogrel was prepared referring to the method in Chinese Journal of Medicinal Chemistry 2007, 17 (3)163-165; prasugrel was prepared referring to the method in Chinese Journal of Pharmaceuticals 2012, 43 (8)647-649; vicagrel was prepared referring to the method in Journal of Medicinal Chemistry, 2012, 55(7), 3342-3352. |
| Subject products: 7 subject products were all provided by Tasly Holding Group. Co. Ltd. | |
| Administration dosage: | |
| | subject products were suspensed in CMC in a concentration of 0.25wt% , were administered in a dosage of 3mg/kg body weight, the administration volume was 2ml. |

### Steps

Animal grouping: the experimental rats were divided randomly according to body weight into negative control group, clopidogrel group, prasugrel group, vicagrel group, TSC-1group, TSC-2 group, TSC-3 group, TSC-4 group, TSC-5 group, TSC-6 group, TSC-7group, TSC-8 group and TSC-9 group, the number of rats n in each group was showed in table 1.

2 hours after administering drugs to the rats, anesthesia with mebubarbital, draw blood from abdominal aorta, anticoagulation with sodium citrate 1:9. Obtained platelet-rich plasma and platelet-poor plasma by centrifugation, the volume ratio of the two was platelet-poor plasma : platelet-rich plasma=3:1.

### Results

**Table 1 The effect of the compound of the present invention on the maximum aggregation rate of platelet aggregation induced by ADP.**

| Group | Administration dosage mg/kg | n | The maximum aggregation rate of platelet irreversible condensed phase |
|---|---|---|---|
| Negative control group | -- | 5 | 61.22±4.73 |
| Clopidogrel group | 3 | 5 | 46.77±8.28* |
| Prasugrel group | 3 | 3 | 20.72±18.84* |
| Vicagrel group | 3 | 2 | 32.36±5.14* |
| TSC-1 group | 3 | 2 | 45.8±3.55* |
| TSC-2group | 3 | 3 | 41.7±7.43* |
| TSC-3group | 3 | 3 | 38.7±4.27* |
| TSC-4group | 3 | 3 | 46.5±8.16* |
| TSC-5group | 3 | 3 | 39.1±5.66* |
| TSC-6group | 3 | 3 | 29.6±5.33* |
| TSC-7group | 3 | 3 | 39.2±6.16* |
| TSC-8group | 3 | 3 | 32.7±9.21* |
| TSC-9group | 3 | 3 | 25.7±3.25* |

| | | | |
|---|---|---|---|
| *Compared with the normal group, P<0.001. | | | |

In the experiment of platelet aggregation induced by ADP, each subject product has the effect of obviously inhibiting the platelet aggregation, and can reverse the platelet second phase aggregation, causing deaggregation. So, the thienopiperidine derivative and pharmaceutically acceptable salt thereof in the present invention can be effectively used for preventing platelet aggregation.

Platelet is a key constituent in the normal clotting mechanism, and also is an important cause forming pathological thrombus, platelet aggregation is the initiating factor forming intra arterial thrombus, playing a key role in initiation of cardiovascular and cerebrovascular diseases (such as heart failure, apoplexy, unstable angina and so on). The chance of occurrence of cardiovascular and cerebrovascular diseases is reduced, while the probably of thrombosis is reduced by inhibiting platelet aggregation. Therefore, inhibiting platelet aggregation has close correlation with preventing or treating cardiovascular and cerebrovascular diseases.

Therefore, as the thienopiperidine derivative and pharmaceutically acceptable salt thereof in the present invention can be effectively used for inhibiting platelet aggregation, it can be effectively used for preventing or treating various diseases caused by platelet aggregation, including by not limited by cardiovascular and cerebrovascular diseases, such as heart failure, apoplexy, unstable angina and so on.

## Claims

1. A thienopiperidine derivative or pharmaceutically acceptable acid addition salts thereof selected from the group consisting of:

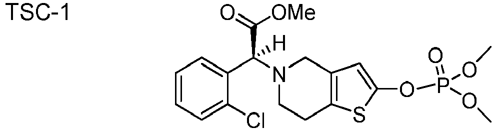

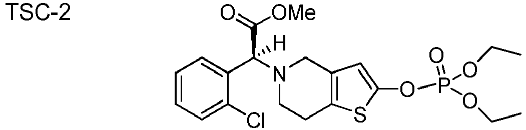

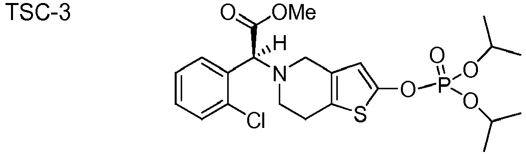

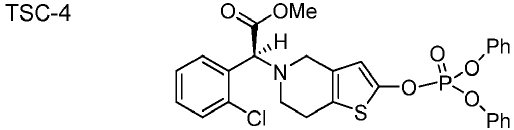

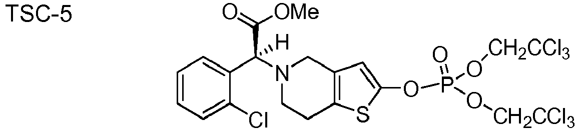

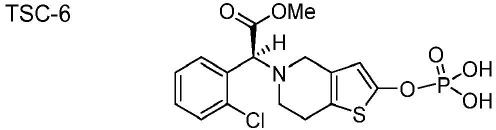

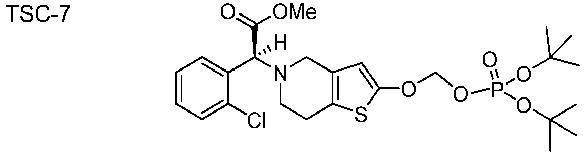

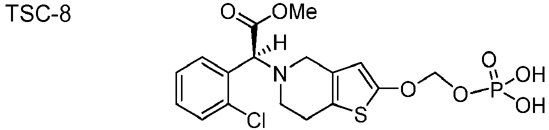

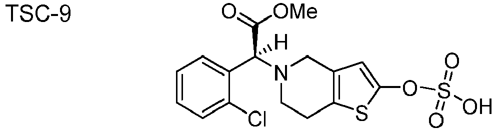

2. The thienopiperidine derivative or pharmaceutically acceptable acid addition salts thereof according to claim 1, wherein said acceptable acid addition salts are prepared by reacting the thienopiperidine derivative with the following acids: sulphuric acid, muriatic acid, hydrobromic acid, phosphoric acid, tartaric acid, fumaric acid, maleic acid, citric acid, acetic acid, formic acid, methanesulfonic acid, p-toluene sulfonic acid, oxalic acid or succinic acid.

3. A pharmaceutical composition containing the thienopiperidine derivative or its pharmaceutically acceptable acid addition salts according to claim 1 or 2.

4. The pharmaceutical composition according to claim 3, wherein said composition further contains pharmaceutically acceptable carrier(s).

5. The thienopiperidine derivative or pharmaceutically acceptable acid addition salts thereof according to claim 1 for use as a medicament.

6. The thienopiperidine derivative or pharmaceutically acceptable acid addition salts thereof according to claim 1 for use in the treatment or preventive treatment of cardiovascular and cerebrovascular diseases.

7. The thienopiperidine derivative or pharmaceutically acceptable acid addition salts thereof for use according to claim 6, wherein said cardiovascular and cerebrovascular diseases are one or more of heart failure, apoplexy and unstable angina.

8. The pharmaceutical composition according to claim 4 for use as a medicament.

9. The pharmaceutical composition according to claim 4 for use in the treatment or preventive treatment of cardiovascular and cerebrovascular diseases.

10. The pharmaceutical composition for use according to claim 9, wherein said cardiovascular and cerebrovascular diseases are one or more of heart failure, apoplexy and unstable angina.

## Patentansprüche

1. Thienopiperidinderivat oder pharmazeutisch akzeptable Säureadditionssalze davon, ausgewählt aus der Gruppe, bestehend aus:

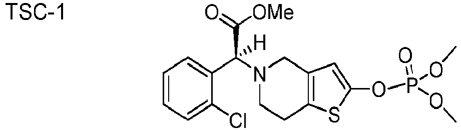

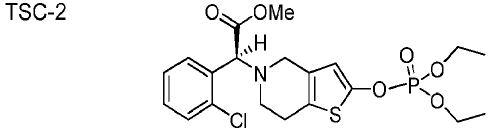

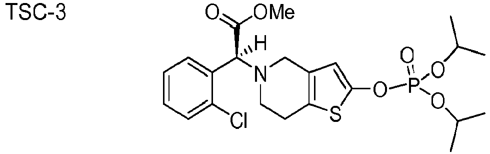

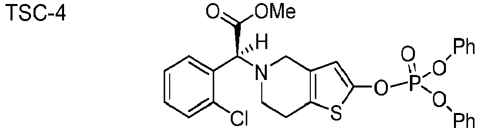

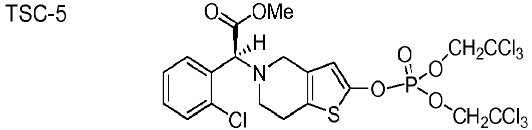

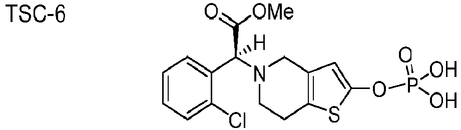

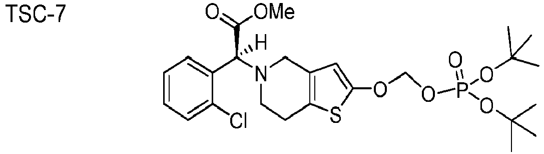

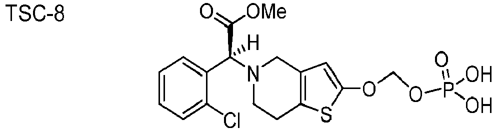

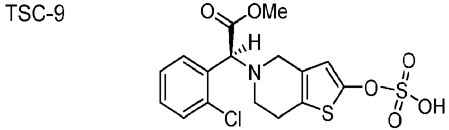

2. Thienopiperidinderivat oder pharmazeutisch akzeptable Säureadditionssalze davon nach Anspruch 1, wobei die akzeptablen Säureadditionssalze durch Zur-Reaktion-Bringen des Thienopiperidinderivats mit den folgenden Säuren hergestellt werden: Schwefelsäure, Salzsäure, Bromwasserstoffsäure, Phosphorsäure, Weinsäure, Fumarsäure, Maleinsäure, Citronensäure, Essigsäure, Ameisensäure, Methansulfonsäure, p-Toluolsulfonsäure, Oxalsäure oder Bernsteinsäure.

3. Pharmazeutische Zusammensetzung, das Thienopiperidinderivat oder seine pharmazeutisch akzeptablen Säureadditionssalze nach Anspruch 1 oder 2 enthaltend.

4. Pharmazeutische Zusammensetzung nach Anspruch 3, wobei die Zusammensetzung ferner pharmazeutisch akzeptable(n) Träger enthält.

5. Thienopiperidinderivat oder pharmazeutisch akzeptable Säureadditionssalze davon nach Anspruch 1 zur Benutzung als ein Medikament.

6. Thienopiperidinderivat oder pharmazeutisch akzeptable Säureadditionssalze davon nach Anspruch 1 zur Benutzung in der Behandlung oder vorbeugenden Behandlung von kardiovaskulären und zerebrovaskulären Erkrankungen.

7. Thienopiperidinderivat oder pharmazeutisch akzeptable Säureadditionssalze davon zur Benutzung nach Anspruch 6, wobei die kardiovaskulären und zerebrovaskulären Erkrankungen eine oder mehrere von Herzinsuffizienz, Apoplexie und instabiler Angina sind.

8. Pharmazeutische Zusammensetzung nach Anspruch 4 zur Benutzung als ein Medikament.

9. Pharmazeutische Zusammensetzung nach Anspruch 4 zur Benutzung in der Behandlung oder vorbeugenden Behandlung von kardiovaskulären und zerebrovaskulären Erkrankungen.

10. Pharmazeutische Zusammensetzung zur Benutzung nach Anspruch 9, wobei die kardiovaskulären und zerebrovaskulären Erkrankungen eine oder mehrere von Herzinsuffizienz, Apoplexie und instabiler Angina sind.

## Revendications

1. Dérivé thiénopipéridine ou ses sels d'addition acide pharmaceutiquement acceptables choisi dans le groupe constitué par :

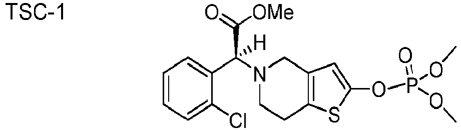

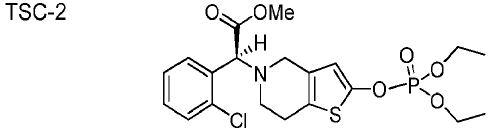

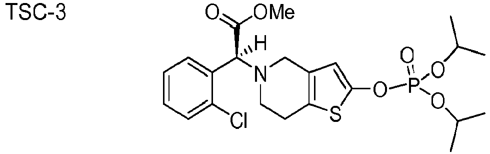

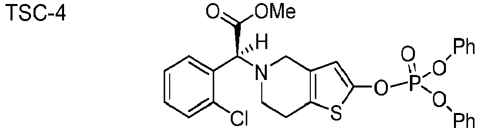

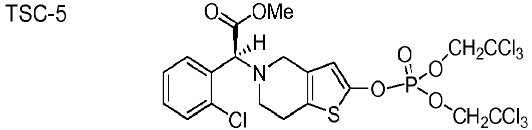

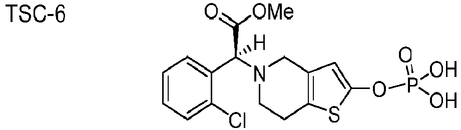

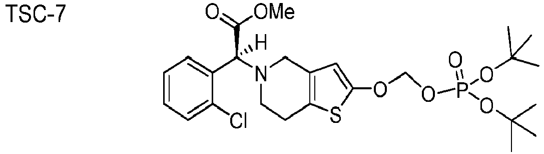

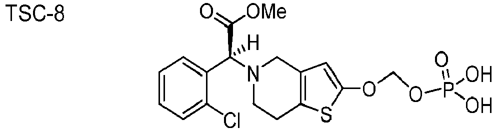

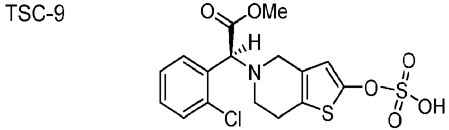

2. Dérivé thiénopipéridine ou ses sels d'addition acide pharmaceutiquement acceptables selon la revendication 1, dans lequel lesdits sels d'addition acide acceptables sont préparés en faisant réagir le dérivé thiénopipéridine avec les acides suivants : l'acide sulfurique, l'acide muriatique, l'acide bromhydrique, l'acide phosphorique, l'acide tartrique, l'acide fumarique, l'acide maléique, l'acide citrique, l'acide acétique, l'acide formique, l'acide méthanesulfonique, l'acide p-toluène sulfonique, l'acide oxalique ou l'acide succinique.

3. Composition pharmaceutique contenant le dérivé thiénopipéridine ou ses sels d'addition acide pharmaceutiquement acceptables selon la revendication 1 ou 2.

4. Composition pharmaceutique selon la revendication 3, dans laquelle ladite composition contient en outre un(des) véhicule(s) pharmaceutiquement acceptable(s).

5. Dérivé thiénopipéridine ou ses sels d'addition acide pharmaceutiquement acceptables selon la revendication 1 destiné à être utilisé comme médicament.

6. Dérivé thiénopipéridine ou ses sels d'addition acide pharmaceutiquement acceptables selon la revendication 1 destiné à être utilisé dans le traitement ou le traitement préventif de maladies cardiovasculaires et cérébrovasculaires.

7. Dérivé thiénopipéridine ou ses sels d'addition acide pharmaceutiquement acceptables destiné à être utilisé selon la revendication 6, dans lequel lesdites maladies cardiovasculaires et cérébrovasculaires sont une ou plusieurs de l'insuffisance cardiaque, de l'apoplexie et de l'angine instable.

8. Composition pharmaceutique selon la revendication 4 destinée à être utilisée comme médicament.

9. Composition pharmaceutique selon la revendication 4 destinée à être utilisée dans le traitement ou le traitement préventif de maladies cardiovasculaires et cérébrovasculaires.

10. Composition pharmaceutique destinée à être utilisée selon la revendication 9, dans laquelle lesdites maladies cardiovasculaires et cérébrovasculaires sont une ou plusieurs de l'insuffisance cardiaque, de l'apoplexie et de l'angine instable.
